# FASCICULE DE BREVET EUROPEEN

(11) **EP 3 142 580 B1**
(45) Date de publication et mention de la délivrance du brevet: **28.02.2018**
(21) Numéro de dépôt: 15720730.9
(22) Date de dépôt: 07.05.2015
(51) Int. Cl.: A61B 17/80, A61B 17/86, A61B 17/70

(54) **ENSEMBLE POUR OSTÉOSYNTHÈSE FORMÉ PAR UNE PLAQUE ET AU MOINS UNE VIS**
ANORDNUNG ZUR OSTEOSYNTHESE AUS EINER PLATTE UND MINDESTENS EINER SCHRAUBE
ASSEMBLY FOR OSTEOSYNTHESIS FORMED BY A PLATE AND AT LEAST ONE SCREW

(30) Priorité: 16.05.2014 FR 1454402
(43) Date de publication de la demande: 22.03.2017
(73) Titulaire: Kisco International, 69800 Saint Priest (FR)
(72) Inventeur: CIRIER, Virgile, F-69300 Caluire et Cuire (FR); HUOT, Jean-Claude, F-64000 Pau (FR); SIMON, Lionel, F-69360 Solaize (FR); VITAL, Jean-Marc, F-33200 Bordeaux (FR)
(74) Mandataire: Lavoix
(86) Numéro de dépôt international: PCT/EP2015/060035
(87) Numéro de publication internationale: WO 2015/173104

(56) Documents cités:
- FR-A1- 2 949 317
- US-A1- 2005 131 413
- US-A1- 2006 276 793
- US-A1- 2007 162 019
- US-B1- 6 322 562

## Description

La présente invention concerne les plaques destinées à l'assemblage de deux dispositifs médicaux, dont une vis, destinés à être implantés sur un os d'un patient lors d'une procédure chirurgicale. Il concerne plus précisément les assemblages vis osseuses-plaques qui sont utilisés en chirurgie de la colonne vertébrale pour stabiliser des vertèbres, notamment lors de l'arthrodèse de vertèbres cervicales.

Lorsqu'une plaque en matériau métallique biocompatible (acier inoxydable, titane...) ou en polymère est utilisée pour stabiliser deux (ou davantage) vertèbres l'une par rapport à l'autre, la fixation de la plaque est assurée au moyen de vis qui peuvent être autotaraudeuses et/ou autoperçantes, allant généralement par paires (deux vis par niveau vertébral) et traversant des orifices correspondants ménagés dans la plaque. Il est évidemment important que ces vis ne puissent pas ressortir progressivement de leur lieu d'implantation en reculant au fur et à mesure de l'utilisation de la plaque, et divers dispositifs anti-recul ont été imaginés à cet effet.

Un dispositif couramment utilisé est l'installation sur la surface de la plaque d'un élément qui vient couvrir la tête d'une ou plusieurs des vis, et qui est lui-même solidarisé à la plaque. Ce dispositif a l'inconvénient de nécessiter des éléments supplémentaires en plus de la plaque et des vis, donc de rallonger la durée de la pose du dispositif et de la rendre plus complexe, de même que son enlèvement lorsque celui-ci est nécessaire pour remplacer un élément du dispositif. Et, l'épaisseur de l'ensemble peut s'en trouver augmentée, ce qui est un inconvénient, en particulier dans la région cervicale.

D'autres dispositifs existent, dans lesquels une pièce additionnelle n'est pas nécessaire, et où la vis est rendue solidaire de la plaque par un bord déformable dudit orifice qui, lors du passage de la vis, prend une forme telle que le retrait spontané de la vis devient impossible. Mais cette déformation est irréversible et rend la plaque inutilisable déjà dans le cas où une seule vis devrait être remplacée.

Une autre fonctionnalité qui doit être présente de façon préférée sur de telles plaques est la possibilité de conférer aisément à l'axe longitudinal de chaque vis une orientation choisie, pouvant être différente d'une orientation qui serait simplement confondue avec celle de l'axe longitudinal de l'orifice correspondant et/ou qui serait perpendiculaire à l'orientation générale de la plaque si celle-ci est sensiblement plane. Autrement dit, la vis doit pouvoir être du type multi-axial.

Le document US-A-2006/276793 décrit des ensembles plaque-vis qui sont conçus pour empêcher un retrait spontané de la vis après la fixation de l'ensemble sur un os (vertèbre notamment), tout en autorisant une rotation relative des vis et de la plaque pour régler leurs orientations, et ce sans nécessiter d'éléments de blocage auxiliaires. Seules les caractéristiques respectives des orifices des plaques et des vis permettent d'obtenir cet effet anti-recul. Dans une première variante, les vis sont équipées de languettes flexibles ou élastiques qui se rétractent lors du passage de la zone la plus étroite de l'orifice, puis reprennent sensiblement leur forme initiale. Dans une autre variante, la vis présente deux filetages successifs, l'un pour la fixation osseuse, l'autre situé près de la tête et de plus grand diamètre que le premier, mais généralement pas identiques pour assurer la continuité de la progression de la vis dans l'os. Le deuxième filetage s'engage sur un filetage femelle ménagé sur la paroi de l'orifice de la plaque.

Le but de l'invention est de proposer une configuration d'un ensemble vis/plaque qui permette de proposer de façon satisfaisante toutes les fonctionnalités précitées, à savoir une plaque pouvant avoir une faible épaisseur sur toute son étendue une fois fixée sur les vertèbres, la possibilité d'une multi-axialité des vis et l'impossibilité d'un retrait spontané progressif des vis.

A cet effet, l'invention a pour objet un ensemble pour ostéosynthèse formé par une plaque et au moins une vis présentant un diamètre de fond de filet pourvue d'une tête, destinée à maintenir ladite plaque en traversant un orifice de ladite plaque pour sa fixation à un os, ladite plaque présentant autant d'orifices que de vis, caractérisé en ce que :
- au moins un desdits orifices de ladite plaque présente sur au moins une portion de sa périphérie une collerette en métal, céramique ou polymère, au droit duquel le diamètre nominal de l'orifice est réduit jusqu'à une valeur inférieure audit diamètre nominal ;
- ladite vis destinée à traverser ledit orifice présente un filetage conique ou bombé sous tête ;
- la collerette et le fond de filet du filetage sous tête présentent en au moins une section de la vis un serrage d'au moins 0,01 mm si la collerette est en métal ou en céramique et d'au moins 0,05 mm si la collerette est en polymère, de façon à créer un point dur vers la fin de la mise en place de la vis, ledit point dur subsistant après que la vis a été mise en place.

Ladite vis peut présenter une partie lisse entre le filetage sous tête et la tête, le diamètre de ladite partie lisse étant inférieur au diamètre de la collerette et la longueur de ladite partie lisse étant supérieure à la hauteur de la collerette.

Ladite collerette peut présenter une largeur et/ou une épaisseur variables selon son périmètre.

Ladite collerette peut présenter des encoches et/ou des échancrures et/ou des amincissements localisés.

Le filetage sous tête de ladite vis peut présenter un nombre pair de filets.

La localisation du filetage sous tête peut permettre à celui-ci de s'ancrer dans l'os cortical lorsque la vis est en place.

Les dimensions relatives des différentes parties de la vis et de la collerette peuvent autoriser une inclinaison de la vis par rapport à l'axe longitudinal de l'orifice de jusqu'à ± 15°.

Ladite collerette peut être rapportée sur la plaque.

Comme on l'aura compris, l'invention repose sur la présence :
- D'une part, de collerettes très peu déformables présentes sur le bord intérieur de, de préférence, chaque orifice de la plaque dans lequel est inséré une vis ;
- D'autre part d'un filet conique ou bombé ménagé sur la vis correspondante et placé juste en-dessous de la tête de la vis, ou n'étant séparé de la tête de la vis que par une courte portion lisse autorisant une inclinaison de l'axe longitudinal de la vis par rapport à l'axe de l'orifice dans lequel elle est insérée ;
- Et de dimensions relatives de la collerette et du fond du filet sous tête de vis qui créent lors du vissage et du dévissage par le chirurgien un « point dur » qui oblige cette partie de la vis à passer en force au-delà de la collerette qui, à cet effet, se déforme légèrement et temporairement, ce qui fait que la remontée spontanée de la vis lors de l'utilisation de la plaque sera empêchée ; en effet, comme la collerette et le filetage sous tête n'ont pas été significativement endommagés lors du serrage, les conditions de création du point dur subsistent à l'issue du vissage ; le franchissement en sens inverse de ce point dur par le filet sous tête n'est donc possible que si on applique un effort de dévissage volontaire à la vis, sensiblement supérieur à l'effort qui est naturellement exercé sur la collerette lors d'un début de remontée spontanée de la vis.

Pour créer ce point dur, tout en permettant son franchissement lors du vissage sans endommagement significatif de la collerette et/ou de la vis de façon à le faire subsister après le vissage complet, le diamètre intérieur de la collerette et le diamètre maximum du fond du filet sous tête ont un serrage d'au moins 0,01 mm lorsque la collerette est en métal ou en céramique, et d'au moins 0,05 mm lorsque la collerette est en polymère. Egalement, le diamètre extérieur du filet sous tête doit être supérieur d'au moins 0,05 mm au diamètre intérieur de la collerette lorsque celle-ci est en métal, et d'au moins 0,1 mm lorsque celle-ci est en polymère. On estime qu'il ne faut pas descendre en-dessous de ces valeurs pour créer un point dur suffisamment efficace.

A l'inverse, il faut aussi que ce serrage ne soit pas trop important, pour éviter une déformation par écrasement irréversible de la vis et/ou de la collerette au point qu'après le vissage, l'ouverture de la collerette et le filet sous tête se retrouveraient avec des dimensions sensiblement modifiées qui feraient que, lors d'une remontée de la vis, celle-ci ne rencontrerait plus assez de résistance de la part de la collerette. Un serrage maximal de l'ordre de 1 mm pourrait être à conseiller dans beaucoup de cas, mais la valeur maximale précise du serrage tolérable est très dépendante des natures des matériaux respectifs de la vis et de la collerette, ainsi que de la forme de la collerette qui contribue à déterminer sa capacité de déformation réversible ou non lors du passage du point dur pendant le vissage. Il sera aisé de déterminer expérimentalement quel serrage maximal serait tolérable à cet effet, pour des morphologies de collerette et des matériaux de vis et de collerette donnés.

Il doit être compris que par « serrage », on entend la différence entre l'ouverture minimale de la collerette et le diamètre maximal de la vis au niveau du fond de filet du filetage sous tête, celui-ci étant supérieur à celle-là d'une valeur égale audit serrage.

Bien entendu, dans le cas le plus général où la collerette est ménagée directement sur la plaque elle-même, le matériau de la collerette et le matériau de la plaque seront le même. Cependant, on peut envisager de prévoir des matériaux différents pour l'une et l'autre si la collerette est ménagée à l'aide d'un élément séparé rapporté sur la plaque (par exemple par une insertion latérale de l'élément à travers une fente traversant la plaque et débouchant dans l'orifice), comme cela serait envisageable. Cette possibilité de prévoir une collerette non venue de matière avec la plaque donne plus de souplesse au chirurgien pour le choix des éléments de l'ensemble et de leurs dimensions. Il faudra cependant veiller à ce que ce choix technologique ne conduise pas à augmenter l'épaisseur de la plaque d'une façon qui la rendrait peu intéressante à utiliser dans la région du rachis où elle est destinée à être implantée, en particulier dans la région cervicale.

En tout état de cause, le diamètre du fond du filet 10 hors de la zone portant le filet sous tête est inférieur au diamètre intérieur de la collerette, de façon à permettre à la vis de traverser sans difficultés la collerette jusqu'à ce que celle-ci rencontre le filet sous tête.

En jouant sur la géométrie de la plaque, il est possible, de manière connue, de prévoir que la vis pénètre dans l'os selon une direction présentant une angulation avec l'axe longitudinal de l'orifice. Autrement dit, une configuration polyaxiale de l'ensemble vis/plaque est possible en utilisant l'invention. A cet effet, on peut prévoir entre le filetage sous tête et la tête une zone dépourvue de filetage et dont le diamètre est inférieur à celui de l'ouverture de la collerette. De cette façon, l'angulation de la vis par rapport à l'axe de l'orifice est toujours possible avant le serrage final et des micromouvements de la vis sont possibles après le serrage.

La dimension du point dur peut être mieux maîtrisée si la vis présente un nombre pair de filets sous tête puisqu'un nombre pair permet le contrôle du diamètre obtenu par deux filets opposés. De tels filets permettent d'entrainer la vis à travers la plaque par le couple de serrage uniquement puisqu'ils viennent en prise sur la collerette comme une vis dans un écrou. Le passage du point dur lors du vissage se fait donc sans qu'il soit nécessaire d'effectuer un effort sur l'os. Cette caractéristique permet également d'exercer une pression symétrique entre la vis et la collerette par rapport à l'axe de la vis, ce qui préserve l'os de contraintes supplémentaires pendant le vissage lors du passage du point dur puisque l'axe de progression de la vis est, de ce fait, invariable, en particulier dans le cas où l'axe de la vis est incliné par rapport à l'axe longitudinal de l'orifice.

Dans un exemple de réalisation, la collerette parcourt la totalité du périmètre de l'orifice de la plaque, avec une largeur et une épaisseur constantes. Mais il est aussi possible de ne ménager cette collerette que sur une partie de ce périmètre, c'est-à-dire de prévoir sur la collerette des encoches, des échancrures ou des amincissements localisés en largeur et/ou en épaisseur. En combinaison avec le choix des matières et des dimensions des pièces, la géométrie de ces encoches/échancrures/amincissements donne une liberté supplémentaire pour la détermination de l'effort nécessaire pour faire passer le point dur à la vis. On peut prévoir, par exemple, au moins deux encoches de chacune 0,5 mm de large. De préférence, en général, la moitié au moins du périmètre de l'orifice devrait présenter une collerette dimensionnée de façon à constituer le point dur dans les conditions selon l'invention.

On peut prévoir de situer le filetage conique ou bombé sous tête à une distance de la tête et sur une longueur telles que, après avoir passé la collerette, ce filetage pénètre dans l'os cortical. Les caractéristiques dimensionnelles du filetage conique ou bombé sont alors particulièrement adaptées à cet effet.

Bien entendu, il est préférable que si la plaque présente plusieurs orifices, chacun d'entre eux soit pourvu d'une collerette selon l'invention et soit traversé par une vis conçue également selon l'invention. Il demeurerait cependant dans l'esprit de l'invention de prévoir que seulement certains des orifices soient pourvus d'une telle collerette et d'une telle vis.

L'invention sera mieux comprise à la lecture de la description qui suit, donnée en référence aux figures annexées suivantes :
- La figure 1 qui montre un exemple de plaque cervicale selon l'invention, avec une vis associée ;
- La figure 2 qui montre, vue en coupe longitudinale selon II-II, une portion de cette plaque, à savoir un des orifices dans lesquels une vis est destinée à être insérée, ainsi que la partie supérieure de la vis correspondante ;
- La figure 3 qui montre une section transversale selon III-III de ladite vis au niveau du filetage conique et/ou bombé qui, selon l'invention, y est pratiqué sous la tête.
- La figure 4 qui montre un autre exemple de plaque cervicale selon l'invention, avec une vis associée, les collerettes de la plaque étant munies d'encoches.

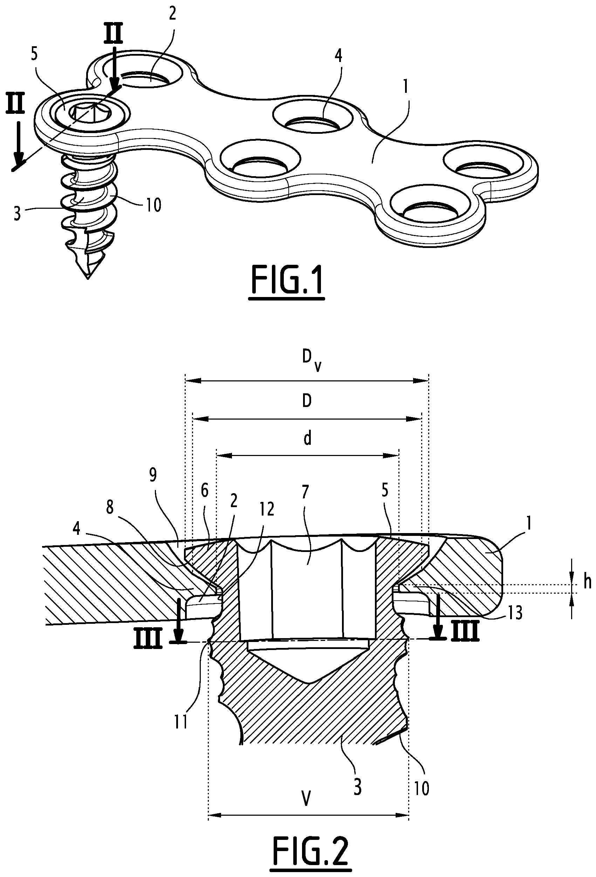

La plaque 1 selon l'invention est, dans l'exemple non limitatif représenté sur la figure 1, d'un type destiné à s'étendre sur trois vertèbres cervicales. Elle présente donc trois paires d'orifices 2, chacun étant destiné à être traversé par une vis 3 (dont une seule est représentée sur la figure 1) qui doit pénétrer dans une vertèbre (en l'occurrence une vertèbre cervicale).

Comme on le voit mieux sur la figure 2, la plaque 1 comporte selon l'invention, sur au moins une des parties de la périphérie de chacun de ses orifices 2, une collerette 4 qui réduit progressivement sur son épaisseur h le diamètre nominal D de l'orifice 2 jusqu'à une valeur minimale d.

La vis 3 selon l'invention présente une tête 5 dont la configuration est classique, en ce qu'elle présente sur sa face supérieure 6 de diamètre Dv un logement 7 de périmètre, par exemple, hexagonal, pour la mise en prise d'un instrument tel qu'un tournevis, et en ce que sa face inférieure présente une portée 8 destinée à coopérer avec une portée correspondante 9 de l'orifice 2 de la plaque 1. Sur l'essentiel de sa longueur, la vis 3 présente un filetage 10 pour son ancrage dans l'os de la vertèbre correspondante. Le diamètre de fond de filet de ce filetage 10 est inférieur au diamètre nominal D de l'orifice 2 et, dans l'exemple représenté, aussi au diamètre d défini par la collerette 4. Ainsi, la vis 3 peut traverser la collerette 4 de l'orifice 2 sans difficultés

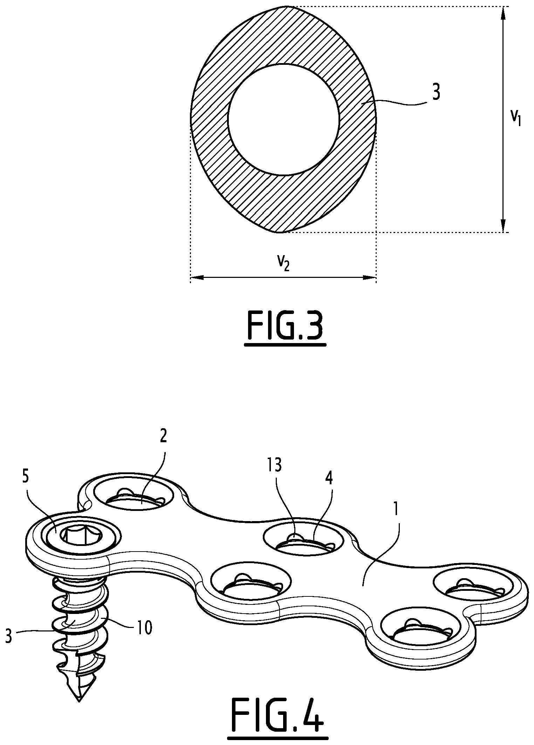

Selon l'invention, sous la tête 5 de la vis 3, se trouve un autre filetage 11 de forme conique ou bombée, dont le diamètre de fond de filet v₁ présente au moins en une section de la vis une valeur supérieure à d de façon à créer un point dur lorsque le filetage sous tête 11 vient au contact de la collerette 4. Le diamètre noté v₂ sur la figure 3, inférieur à v₁, correspond à la trace de l'outil qui crée une zone de moindre diamètre du filet sous tête 11.

Comme on l'a dit, (v₁-d) est supérieur ou égal à 0,01 mm lorsque la collerette 4 est en métal ou en céramique et supérieur ou égal à 0,05 mm lorsque la collerette 4 est en polymère.

On appelle V le diamètre maximal du filet sous tête 11. V définit une butée axiale de la vis sur la collerette de diamètre d de manière à empêcher le recul intempestif de la vis. Le filetage sous tête 11 se prend dans la collerette comme une vis dans un écrou lors de la progression de la vis 3, et à la faveur du couple de serrage ou de desserrage, il permet d'entrainer la vis dans ou hors de la plaque sans contrainte sur l'os. Mais il ne peut franchir le point dur que si un couple suffisant est délibérément appliqué à la vis 3 par le chirurgien, de sorte qu'un retrait spontané de la vis 3 entraînant un passage non désiré du point dur, donc un retrait spontané de la vis hors de l'os, n'est pas possible.

Le filetage sous tête 11 a, dans l'exemple représenté, comme c'est préférable, un nombre pair de filets, en l'occurrence deux, pour les raisons qui ont été dites.

Le filetage sous tête 11 est, de préférence et comme représenté, présent sur une localisation qui lui permet de se retrouver dans l'os cortical de la vertèbre après le serrage de la vis 3. A cet effet, une augmentation du diamètre de fond de filet favorise la compression osseuse, et une augmentation du nombre de filets favorise l'accroche de la vis dans l'os cortical.

Le pas du filetage sous tête 11 est de préférence égal à ou proche de celui du filetage à os 10, Il exerce ainsi peu d'effort axial sur l'os au passage du point dur. Par exemple, les deux pas ne diffèrent que de 0,2 mm.

Quant au restant du filetage de la vis 4, il est adapté pour se fixer au mieux dans l'os, comme c'est classiquement le cas.

Selon une variante préférée de l'invention, entre le filetage sous tête 11 et la tête 5 de la vis 3 se trouve une partie lisse 12 dont la longueur est, de préférence, supérieure à la hauteur h de la collerette 4, comprise par exemple entre 0,3 et 0,5 mm, et de diamètre inférieur à d. Elle permet de libérer le filet 11 de la plaque et ainsi d'assurer le contact entre la tête de vis et la plaque suivant l'inclinaison choisie par le chirurgien, dans l'intervalle d'inclinaison défini par son diamètre, par exemple un maximum de jusqu'à ± 15° par rapport à l'axe longitudinal du trou de la plaque. Ainsi le serrage de la plaque à l'os à l'aide de la vis, s'effectue sans autre effort entre la plaque et la vis que l'effort souhaité dans l'axe de la vis.

Comme on le voit sur la figure 4, dans l'exemple représenté on peut prévoir sur la collerette 4 des échancrures 13, qui sont ici de forme demi-circulaire. Il est préférable que ces encoches ne soient pas diamétralement opposées pour ne pas altérer le fonctionnement du point dur. Par exemple, on peut prévoir trois encoches réparties à 120° l'une de l'autre.

A titre d'exemple, on peut prévoir une plaque 1 en titane et une vis 3 également en titane, la plaque 1 ayant des orifices 2 de diamètre D de 5 mm, des collerettes de diamètre d de 3,9 mm, et la vis 3 ayant un diamètre maximal de tête Dv de 5,3 mm et un filetage sous tête 11 de diamètre maximal V de 4,3 mm et de diamètre de fond de filet v₁ de 4 mm.

Comme on le voit dans l'exemple représenté, il est possible, en dimensionnant correctement les différentes parties de la plaque et de la vis, de faire en sorte que la tête de la vis ne dépasse pas de la face supérieure de la plaque 1. Cela rend l'invention particulièrement bien adaptée aux plaques cervicales dont l'épaisseur totale doit être aussi réduite que possible pour qu'elles puissent être opérationnelles dans leur environnement.

On a décrit l'invention en référence à un ensemble plaque cervicale-vis, mais il est évident que l'invention peut s'appliquer à tous types de dispositifs d'ostéosynthèse comportant une plaque traversée par une ou des vis dont il est souhaitable d'empêcher qu'elles puissent sortir progressivement de leur logement. L'invention a aussi pour avantage notable de ne nécessiter à cet effet aucune pièce additionnelle qui serait susceptible d'allonger le temps de pose du dispositif et d'être mal mise en place pendant cette pose, ou d'être perdue, ou qui confèrerait à l'ensemble qu'elle formerait avec la plaque et les vis une épaisseur supérieure à celle qui la plaque et les têtes des vis imposeraient seules.

## Revendications

1. Ensemble pour ostéosynthèse formé par une plaque (1) et au moins une vis (3) pourvue d'une tête (5), et d'un filetage sous tête (11) présentant un diamètre de fond de filet (v₁), destinée à maintenir ladite plaque (1) en traversant un orifice (2) de ladite plaque (1) pour sa fixation à un os, ladite plaque (1) présentant autant d'orifices (2) que de vis (3), au moins un desdits orifices (2) de ladite plaque (1) présente sur au moins une portion de sa périphérie une collerette (4), au droit de laquelle le diamètre nominal (D) de l'orifice (2) est réduit jusqu'à une valeur (d) inférieure audit diamètre nominal, **caractérisé en ce que** :
ladite collerette (4) est en métal, céramique ou polymère ;
- ledit filetage sous tête (11) est conique ou bombé ;
- la collerette (4) et le fond de filet du filetage sous tête (11) présentent en au moins une section de la vis (3) un serrage (v₁ - d) d'au moins 0,01 mm si la collerette (4) est en métal ou en céramique et d'au moins 0,05 mm si la collerette (4) est en polymère, de façon à créer un point dur vers la fin de la mise en place de la vis (3), ledit point dur subsistant après que la vis (3) a été mise en place.

2. Ensemble selon la revendication 1, **caractérisé en ce que** ladite vis (3) présente une partie lisse (12) entre le filetage sous tête (11) et la tête (5), le diamètre de ladite partie lisse (12) étant inférieur au diamètre (d) de la collerette (4) et la longueur de ladite partie lisse (12) étant supérieure à la hauteur (h) de la collerette (4).

3. Ensemble selon la revendication 1 ou 2, **caractérisé en ce que** ladite collerette (4) présente une largeur et/ou une épaisseur variables selon son périmètre.

4. Ensemble selon la revendication 3, **caractérisé en ce que** ladite collerette (4) présente des encoches et/ou des échancrures (12) et/ou des amincissements localisés..

5. Ensemble selon l'une des revendications 1 à 4, **caractérisé en ce que** le filetage sous tête (11) de ladite vis (3) présente un nombre pair de filets.

6. Ensemble selon l'une des revendications 1 à 5, **caractérisé en ce que** la localisation du filetage sous tête (11) permet à celui-ci de s'ancrer dans l'os cortical lorsque la vis (3) est en place.

7. Ensemble selon l'une des revendications 2 à 6, **caractérisé en ce que** les dimensions relatives des différentes parties de la vis (3) et de la collerette (4) autorisent une inclinaison de la vis (3) par rapport à l'axe longitudinal de l'orifice (2) de jusqu'à ± 15°.

8. Ensemble selon l'une des revendications 1 à 7, **caractérisé en ce que** ladite collerette (4) est rapportée sur la plaque (1).

## Patentansprüche

1. Osteosynthesebaugruppe, welche aus einer Platte (1) und zumindest einer Schraube (3) gebildet ist, die mit einem Kopf (5) und mit einem einen Kerndurchmesser (v₁) aufweisenden Gewinde (11) unter dem Kopf ausgestattet ist, die dazu bestimmt ist, um die besagte Platte (1) beim Hindurchtreten durch eine Öffnung (2) der besagten Platte (1) zu deren Fixierung an einem Knochen zu halten, wobei die besagte Platte (1) genauso viele Öffnungen (2) wie Schrauben (3) aufweist, wobei zumindest eine der besagten Öffnungen (2) der besagten Platte (1) an zumindest einem Abschnitt ihrer Peripherie einen Flansch (4) aufweist, mit dessen Hilfe der Nominaldurchmesser (D) der Öffnung (2) bis auf einen Wert (d) verringert ist, welcher kleiner ist als der besagte Nominaldurchmesser, **dadurch gekennzeichnet, dass**:
der besagte Flansch (4) aus Metall, Keramik oder einem Polymer ist,
das besagte Gewinde (11) unter dem Kopf konisch oder gewölbt ist,
der Flansch (4) und der Kern des Gewindes (11) unter dem Kopf an zumindest einem Abschnitt der Schraube (3) eine Klemmung (v₁ - d) von zumindest 0,01 mm, wenn der Flansch (4) aus Metall oder aus Keramik ist, und von zumindest 0,05 mm, wenn der Flansch (4) aus Polymer ist, in der Art des Erzeugens einer harten Stelle zum Ende des Einführens der Schraube (3) hin haben, wobei diese harte Stelle fortbesteht, nachdem die Schraube (3) eingeführt worden ist.

2. Baugruppe gemäß dem Anspruch 1, **dadurch gekennzeichnet, dass** die besagte Schraube (3) einen glatten Abschnitt (12) zwischen dem Gewinde (11) unter dem Kopf und dem Kopf (5) aufweist, wobei der Durchmesser des besagten glatten Abschnitts (12) kleiner ist als der Durchmesser (d) des Flanschs (4) und die Länge des besagten glatten Abschnitts (12) größer ist als die Höhe (h) des Flanschs (4).

3. Baugruppe gemäß dem Anspruch 1 oder 2, **dadurch gekennzeichnet, dass** der Flansch (4) entlang seinem Umfang eine variable Breite und/oder eine variable Dicke aufweist.

4. Baugruppe gemäß dem Anspruch 3, **dadurch gekennzeichnet, dass** der besagte Flansch (4) lokale Nuten und/oder Schlitze (12) und/oder Verjüngungen aufweist.

5. Baugruppe gemäß irgendeinem der Ansprüche 1 bis 4, **dadurch gekennzeichnet, dass** das Gewinde (11) unter dem Kopf der besagten Schraube (3) eine gerade Anzahl von Gewindegängen aufweist.

6. Baugruppe gemäß irgendeinem der Ansprüche 1 bis 5, **dadurch gekennzeichnet, dass** die Lage des Gewindes (11) unter dem Kopf es ihm erlaubt, am Kortikalknochen verankert zu sein, wenn die Schraube (3) platziert ist.

7. Baugruppe gemäß irgendeinem der Ansprüche 2 bis 6, **dadurch gekennzeichnet, dass** die Relativabmessungen von verschiedenen Abschnitten der Schraube (3) und des Flanschs (4) eine Neigung der Schraube (3) bezüglich einer Longitudinalachse der Öffnung (2) von bis zu ± 15° erlauben.

8. Baugruppe gemäß irgendeinem der Ansprüche 1 bis 7, **dadurch gekennzeichnet, dass** der besagte Flansch (4) an die Platte (1) angesetzt ist.

## Claims

1. An osteosynthesis assembly formed by a plate (1) and at least one screw (3) provided with a head (5) and an under-head threading (11), designed to maintain said plate (1) by crossing through an orifice (2) of said plate (1) to fasten it to a bone, said plate (1) having as many orifices (2) as there are screws (3), at least one of said orifices (2) of said plate (1) having over at least a portion of its periphery a collar (4), at the level of which the nominal diameter (D) of the orifice is reduced to a value (d) below said nominal diameter, **characterized in that**:
- said collar (4) is made from metal, ceramic or polymer;
- said under-head threading (11) is conical or curved;
- the collar (4) and the thread bottom of the under-head threading (11) have, in at least one section of the screw (3), a tightening (v₁-d) of at least 0.01 mm if the collar (4) is made from metal or ceramic and at least 0.05 mm if the collar (4) is made from polymer, so as to create a hard spot toward the end of the placement of the screw (3), said hard spot remaining after the screw (3) has been placed.

2. The assembly according to claim 1, **characterized in that** said screw (3) has a smooth part (12) between the under-head threading (11) and the head (5), the diameter of said smooth part (12) being smaller than the diameter (d) of the collar (4) and the length of said smooth part (12) being greater than the height (h) of the collar (4).

3. The assembly according to claim 1 or 2, **characterized in that** said collar (4) has a variable width and/or thickness along its perimeter.

4. The assembly according to claim 3, **characterized in that** said collar (4) has notches and/or indentations (12) and/or localized thinner areas.

5. The assembly according to one of claims 1 to 4, **characterized in that** under-head threading (11) of said screw (3) may have an even number of threads.

6. The assembly according to one of claims 1 to 5, **characterized in that** the location of the under-head threading (11) allows the latter to be anchored in the cortical bone when the screw (3) is in place.

7. The assembly according to one of claims 2 to 6, **characterized in that** the relative dimensions of the various parts of the screw (3) and the collar (4) allow an incline of the screw (3) relative to the longitudinal axis of the orifice (2) of up to ± 15°.

8. The assembly according to any one of claims 1 to 7, **characterized in that** said collar (4) is attached on the plate (1).
